# EUROPÄISCHE PATENTSCHRIFT

(11) **EP 3 246 059 B1**
(45) Veröffentlichungstag und Bekanntmachung des Hinweises auf die Patenterteilung: **04.03.2020**
(21) Anmeldenummer: 17170354.9
(22) Anmeldetag: 10.05.2017
(51) Int. Cl.: A61M 5/168, A61M 39/28

(54) **DURCHFLUSSREGELEINRICHTUNG FÜR EINE SCHLAUCHLEITUNG EINES MEDIZINISCHEN INFUSIONSSYSTEMS**
FLOW RATE REGULATING MECHANISM FOR A HOSE OF A MEDICAL INFUSION SYSTEM
DISPOSITIF DE RÉGULATION DE DÉBIT POUR UNE CONDUITE D'UN SYSTÈME D'INFUSION MÉDICAL

(30) Priorität: 19.05.2016 DE 102016208677
(43) Veröffentlichungstag der Anmeldung: 22.11.2017
(73) Patentinhaber: B. Braun Melsungen AG, 34212 Melsungen (DE)
(72) Erfinder: Bolz, Johannes, 34134 Kassel (DE)
(74) Vertreter: Patentanwälte Ruff, Wilhelm, Beier, Dauster & Partner mbB

(56) Entgegenhaltungen:
- WO-A1-2017/149290
- GB-A- 646 167
- US-A- 4 114 640

## Beschreibung

Die Erfindung betrifft eine Durchflussregeleinrichtung für eine Schlauchleitung eines medizinischen Infusionssystems nach dem Oberbegriff des Anspruchs 1.

Eine derartige Durchflussregeleinrichtung ist aus der nicht vorveröffentlichten WO 2017/149290 A1 bekannt.

Eine weitere Durchflussregeleinrichtung ist aus der US 4,114,640 A bekannt. Die bekannte Durchflussregeleinrichtung weist zwei Klemmglieder auf, deren innenliegende Wandungen Konturen bilden, die die Schlauchleitung auf gegenüberliegenden Seiten flankieren. Diese Wandungen sind zumindest abschnittsweise exzentrisch zu einer Drehachse des jeweiligen Klemmgliedes gestaltet. Die beiden Klemmglieder sind manuell verstellbar, um eine Fluiddurchflussrate der Schlauchleitung zu verändern.

Die GB 646,167 A offenbart eine Durchflussregeleinrichtung für Schlauchleitungen mit zwei Rollenklemmeinrichtungen, die von gegenüberliegenden Seiten her dem Außenmantel der Schlauchleitung zustellbar sind.

Die US 8 544 815 B2 offenbart eine Durchflussregeleinrichtung für eine Schlauchleitung eines medizinischen Infusionssystems. Die Durchflussregeleinrichtung weist ein Gehäuse auf, in dem eine Kanalführung für einen Schlauchabschnitt der Schlauchleitung vorgesehen ist. Der Kanalführung ist ein Klemmsegment zugeordnet, das bogenförmig an die Kanalführung angepasst ist und ein großflächiges Pressen des in der Kanalführung befindlichen Schlauchabschnittes ermöglicht. Eine Verlagerung des Klemmsegmentes erfolgt durch einen in dem Gehäuse drehbeweglich gelagerten Exzenter. Der Exzenter wird über ein Stellrad verdreht, das außenseitig am Gehäuse gelagert ist. Um das Klemmsegment zwischen einer Freigabestellung und einer den Schlauchabschnitt für eine Sperrstellung zusammenpressenden Schließstellung zu verlagern, muss das Stellrad um wenigstens 180° verdreht werden.

Aufgabe der Erfindung ist es, eine Durchflussregeleinrichtung der eingangs genannten Art zu schaffen, die mit geringem Aufwand herstellbar und einfach bedienbar ist sowie eine genaue Einstellbarkeit einer Tropfrate ermöglicht.

Diese Aufgabe wird durch die Merkmale des Anspruchs 1 gelöst. Dadurch, dass der jeweilige Schlauchabschnitt zwischen zwei Klemmgliedern erstreckt ist, ist eine symmetrische Pressung des Schlauchabschnittes von gegenüberliegenden Seiten her ermöglicht. Hierdurch ergibt sich eine verbesserte Einstellbarkeit einer Durchflussrate der Schlauchleitung und demzufolge einer Tropfrate. Zudem wird der Mantel des Schlauchabschnittes gleichmäßig und in geringerem Maße deformiert als bei einer einseitigen Belastung. Die erfindungsgemäße Lösung ermöglicht eine Linienpressung des Schlauchabschnittes, da der Schlauchabschnitt nach Art einer Tangente zwischen gegenüberliegenden Krümmungsscheitelpunkten der beiden Klemmglieder hindurchgeführt wird. Die Erfindung ist vorteilhaft für schwerkraftbetriebene Infusionssysteme einsetzbar. Erfindungsgemäß ist ein exzentrischer Klemmabschnitt der Umfangskontur konkav ausgeführt mit einem radialen sowie einem axialen Stützwandungsbereich, die den Schlauchabschnitt relativ zu einer Drehachse des Klemmgliedes mantelseitig beaufschlagen abhängig von einer Drehung des Klemmgliedes. Die Begriffe radial und axial sind auf die Drehachse des Klemmgliedes bezogen und bedeuten, dass der entsprechende Schlauchabschnitt im Querschnitt gesehen etwa rechtwinklig umschlossen ist, um ein Ausweichen des Schlauchabschnitts in Axialrichtung der Drehachse des Klemmgliedes und in Radialrichtung der Drehachse des Klemmgliedes zu unterbinden.

Erfindungsgemäß sind die beiden Klemmglieder mittels einer Synchronisiereinrichtung synchron gegenläufig zueinander drehbeweglich gelagert. Vorzugsweise rollen die gegenüberliegenden Klemmglieder über ihre Synchronisiereinrichtung gegenläufig aufeinander ab. Als Synchronisiereinrichtung ist vorzugsweise ein Verzahnungsgetriebe vorgesehen, wobei jedem Klemmglied jeweils ein komplementärer Verzahnungsabschnitt zugeordnet ist. Vorzugsweise sind die Verzahnungsabschnitte koaxial zu den Drehachsen der Klemmglieder angeordnet.

Erfindungsgemäß weist die Synchronisiereinrichtung zwei miteinander kämmende, kreisbogenförmige Verzahnungsabschnitte auf, die jeweils einem Klemmglied zugeordnet sind. Die Verzahnungsabschnitte sind koaxial zu der jeweiligen Drehachse ausgerichtet. Die Verzahnungsabschnitte sind identisch zueinander ausgeführt, um ein spielfreies Kämmen nach Art von zwei Zahnrädern zu bewirken. Vorzugsweise erstrecken sich die Verzahnungsabschnitte jeweils über einen Kreisbogen, der einen Winkel von maximal 90° überstreicht.

In Ausgestaltung der Erfindung sind die Verzahnungsabschnitte einstückig an den Klemmgliedern vorgesehen. Dabei ist jeweils ein Verzahnungsabschnitt dem zugehörigen Klemmglied zugeordnet. Vorzugsweise sind die beiden Klemmglieder aus Kunststoff hergestellt und die Verzahnungsabschnitte sind einstückig an den Klemmgliedern angeformt.

In weiterer Ausgestaltung der Erfindung ist ein Verstellwinkel jedes Klemmgliedes zwischen einer den Schlauchabschnitt für einen vollen Fluiddurchfluss freigebenden Öffnungsstellung und einer den Schlauchabschnitt für einen Fluiddurchfluss verschließenden Sperrstellung kleiner oder gleich 90°. Dieser relativ kleine Verstellweg ermöglicht eine äußerst einfache und ergonomisch günstige manuelle Bedienung. Mit einem Handgriff kann eine Verstellung zwischen der Öffnungsstellung und der Sperrstellung vorgenommen werden. Vorzugsweise beträgt auch ein durch den jeweiligen kreisbogenförmigen Verzahnungsabschnitt überstrichener Winkel maximal 90°. Bei einstückiger Anformung des jeweiligen Verzahnungsabschnittes an das jeweilige Klemmglied sind der Verstellwinkel des Klemmgliedes und der überstrichene Winkel des jeweiligen Verzahnungsabschnittes zwangsläufig identisch.

In weiterer Ausgestaltung der Erfindung sind die beiden Klemmglieder relativ zueinander gelagert und gehalten. Die beiden Klemmglieder sind entweder direkt miteinander verbunden und demzufolge relativ zueinander gelagert und gehalten, oder sie sind in einem Trägergehäuse drehbar gelagert, das vorzugsweise ebenfalls aus Kunststoff hergestellt ist.

In weiterer Ausgestaltung der Erfindung ist das Trägergehäuse aus einer Bodenschale und einer Deckschale aufgebaut, zwischen denen die Klemmglieder angeordnet sind, wobei die Bodenschale und die Deckschale im Bereich der Drehachsen miteinander verbunden sind. Hierdurch ergibt sich eine besonders einfache Herstellbarkeit der Durchflussregeleinrichtung. Die Durchflussregeleinrichtung kann aus wenigen Kunststoffbauteilen zusammengebaut werden. Die Bodenschale und die Deckschale können als Gleichteile gestaltet sein.

In weiterer Ausgestaltung der Erfindung sind die beiden Klemmglieder als Gleichteile gestaltet. Hierdurch wird eine weitere Vereinfachung der Herstellung und demzufolge eine Kostenreduzierung erzielt.

In weiterer Ausgestaltung der Erfindung weist jedes Klemmglied wenigstens einen Bedienhebel auf, der gegenüberliegend zu dem exzentrischen Klemmabschnitt relativ zu der Drehachse nach außen abragt. Der jeweilige Bedienhebel ist derart gegenüberliegend positioniert, dass er relativ zur Drehachse einen Hebelarm für die Verdrehung des exzentrischen Klemmabschnittes bewirkt. In besonders vorteilhafter Weise ist der Bedienhebel einstückig am Klemmglied angeformt. Der Bedienhebel ragt in vorteilhafter Weise flügelartig ab, so dass die Bedienhebel der beiden Klemmglieder nach Art von Flügelschenkeln nach außen abragen und gemeinsam manuell bedienbar sind.

In weiterer Ausgestaltung der Erfindung weist jedes Klemmglied zwei Bedienhebel auf, die wippenartig in unterschiedlichen Richtungen zu der Drehachse abragen. Alle Bedienhebel ragen in der Drehebene des jeweiligen Klemmgliedes nach außen ab. Durch die wippenartige Ausrichtung ist gewährleistet, dass wenigstens ein Bedienhebel jedes Klemmgliedes für eine manuelle Bedienung unabhängig von der Stellung des jeweiligen Klemmgliedes immer für die Bedienperson gut zugänglich ist.

In weiterer Ausgestaltung der Erfindung ragt der wenigstens eine Bedienhebel zumindest abschnittsweise seitlich nach außen über das Trägergehäuse hinaus. Hierdurch wird die manuelle Bedienbarkeit der Durchflussregeleinrichtung weiter verbessert. Denn dadurch, dass der wenigstens eine Bedienhebel seitlich nach außen hinausragt, ist der wenigstens eine Bedienhebel für eine Bedienperson immer gut erkennbar und ergonomisch einfach betätigbar.

Weitere Vorteile und Merkmale der Erfindung ergeben sich aus den Ansprüchen. Nachfolgend sind bevorzugte Ausführungsbeispiele der Erfindung beschrieben und anhand der Zeichnungen dargestellt.
- Fig. 1 bis 3: zeigen in unterschiedlichen Stellungen ein schematisches Funktionsprinzip einer Ausführungsform einer erfindungsgemäßen Durchflussregeleinrichtung,
- Fig. 4 bis 7: in einer Frontansicht eine praktische Ausführungsform einer erfindungsgemäßen Durchflussregeleinrichtung in unterschiedlichen Betätigungsstellungen,
- Fig. 8 bis 11: in Längsschnittdarstellungen die Durchflussregeleinrichtung nach den Fig. 4 bis 7 in den unterschiedlichen Betätigungsstellungen und
- Fig. 12 bis 15: jeweils in einer Draufsicht die Durchflussregeleinrichtung nach den Fig. 4 bis 11 in den unterschiedlichen Betätigungsstellungen analog der Fig. 8 bis 11.

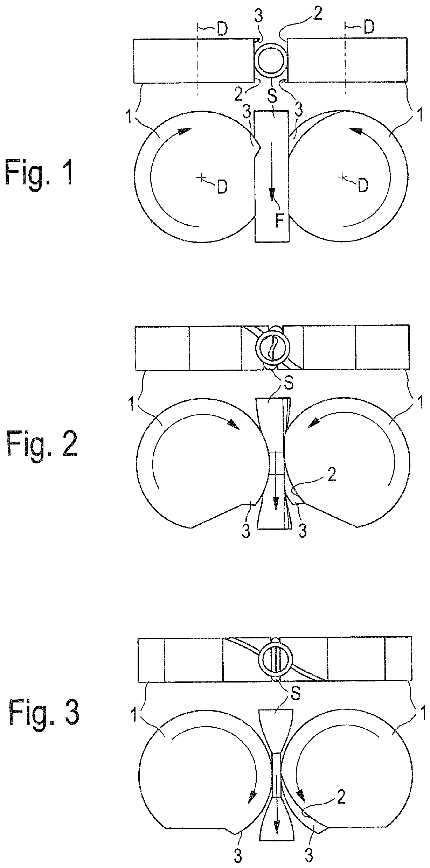

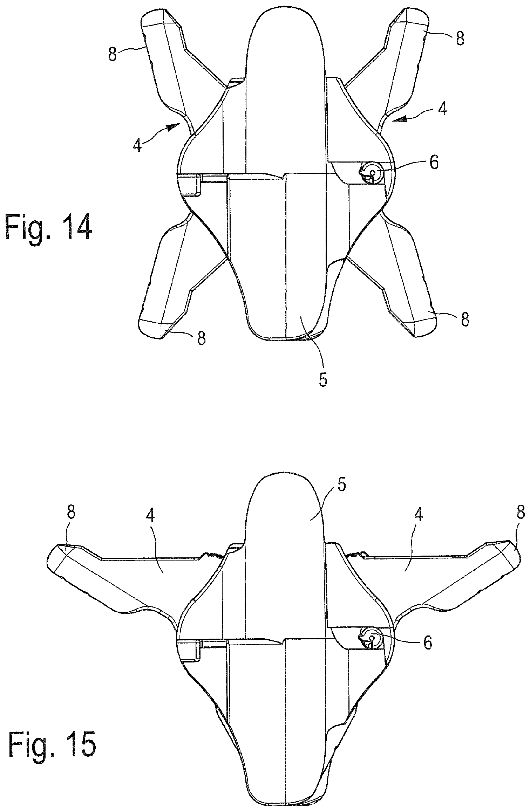

Eine Durchflussregeleinrichtung, wie sie anhand der Fig. 1 bis 15 dargestellt ist, ist für den Einsatz bei einer Schlauchleitung eines medizinischen Infusionssystems vorgesehen, wobei das Infusionssystem schwerkraftbetrieben ist. Die Schlauchleitung dient dazu, ein Fluid aus einem Fluidspeicher einem Katheter zuzuführen, der patientenseitig befestigbar ist. Die Schlauchleitung ist flexibel gestaltet und weist einen Schlauchabschnitt S auf, der in montiertem Zustand innerhalb der Durchflussregeleinrichtung gemäß den Fig. 1 bis 15 erstreckt ist. Mittels der Durchflussregeleinrichtung kann der Schlauchabschnitt S stufenlos von einer Öffnungsstellung, in der ein freier Querschnitt des Schlauchabschnittes S unbelastet ist, bis zu einer Sperrstellung, in der der Schlauchabschnitt von gegenüberliegenden Seiten her vollständig zusammengepresst ist, beaufschlagt werden. Hierdurch ist eine Durchflussrate des durch den Schlauchabschnitt S hindurchströmenden Fluids einstellbar. Der Schlauchabschnitt S der Schlauchleitung ist zwischen zwei drehbeweglich gelagerten Klemmgliedern 1 der Durchflussregeleinrichtung hindurchgeführt, die relativ zueinander oder relativ zu einem Trägergehäuse um jeweils eine Drehachse D drehbar gelagert sind. Die beiden Drehachsen D sind parallel zueinander ausgerichtet. Die beiden Klemmglieder 1 sind gemäß der schematischen Ausführungsform nach den Fig. 1 bis 3 als drehbare Zylinderkörper ausgeführt, die den Schlauchabschnitt S auf gegenüberliegenden Seiten flankieren. Die beiden Klemmglieder 1 sind in einer gemeinsamen Ebene drehbar gelagert, in der auch der Schlauchabschnitt S erstreckt ist. Diese gemeinsame Ebene bildet eine gemeinsame Radialebene der beiden Drehachsen D. Die beiden Klemmglieder 1 weisen jeweils eine Umfangskontur im Bereich ihres Umfangsrandes auf, die über einen Teilbereich ihres Umfangs jeweils einen exzentrischen Klemmabschnitt 2, 3 aufweisen, dessen Abstand zur jeweiligen Drehachse D sich über die in Umfangsrichtung gesehene Länge des Klemmabschnittes kontinuierlich ändert. Die beiden Klemmglieder 1 mit ihren exzentrischen Klemmabschnitten 2, 3 sind identisch zueinander gestaltet, wobei jedoch das eine Klemmglied 1 gegenüber dem anderen Klemmglied 1 um 180° verdreht ausgerichtet ist. Die Drehachsen D der beiden zylinderartigen Klemmglieder 1 liegen - in der Draufsicht gesehen - auf einer Verbindungslinie, die eine Längsrichtung des Schlauchabschnittes S (symbolisiert durch eine Fluiddurchflussrichtung F) orthogonal schneidet. Damit wirken auch entsprechende Kräfte der Anlagekonturen der exzentrischen Klemmabschnitte 2, 3 im Bereich dieser Verbindungslinie auf entsprechend gegenüberliegende Mantelaußenflächen des Schlauchabschnittes S. Rückstellkräfte des Schlauchabschnittes S, die durch elastische Deformationskräfte bei einem Zusammenpressen des Schlauchabschnittes S entstehen, wirken demzufolge entlang dieser imaginären Verbindungslinie exakt in Richtung der Drehachsen D.

Die entsprechenden Anlagekonturen der beiden exzentrischen Klemmabschnitte der gegenüberliegenden Klemmglieder 1 weisen sowohl radiale Stützwandungsbereiche 2 als auch axiale Stützwandungsbereiche auf. Die radialen und axialen Stützwandungsbereiche gehen über die Länge einer Abwicklung der Umfangskontur des jeweiligen Klemmgliedes 1 gesehen kontinuierlich konisch und/oder konkav ineinander über. Maßgeblich ist es, dass die entsprechenden Anlagekonturen auch bei geneigter, konischer, konkaver oder konvexer Krümmung immer axiale und radiale Stützkomponenten bilden, die auf den Schlauchabschnitt S wirken.

Anhand der Fig. 2 ist erkennbar, dass die gegenüberliegenden Anlagekonturen der beiden exzentrischen Klemmabschnitte der Klemmglieder 1 einander relativ zu einer Diagonalen derart spiegelsymmetrisch gegenüberliegen, dass sie in bestimmten Quetschstellungen des Schlauchabschnittes S mittige Linienberührungen einer Innenseite des Mantels des Schlauchabschnittes S bewirken, wodurch sich oberhalb und unterhalb dieser Linienberührungen zwei voneinander getrennte Fluidkanalabschnitte des Schlauchabschnittes S bilden. Bei vollständig zusammengepresstem Schlauchabschnitt S ist ein Fluiddurchfluss komplett gesperrt, wie Fig. 3 zu entnehmen ist. In dieser Stellung haben die exzentrischen Klemmabschnitte der Klemmglieder 1 keine wirksamen axialen Stützwandungsbereiche mehr. Vielmehr sind die entsprechenden Anlagekonturen ausschließlich zur radialen Abstützung vorgesehen, wie Fig. 3 zu entnehmen ist.

Um zu gewährleisten, dass die beiden Klemmglieder 1 immer synchron und gegenläufig zueinander verdreht werden, weist die Durchflussregeleinrichtung zusätzlich eine Synchronisiereinrichtung auf, die auf die beiden Klemmglieder 1 wirkt. In den Fig. 1 bis 3 ist diese Synchronisiereinrichtung nicht dargestellt.

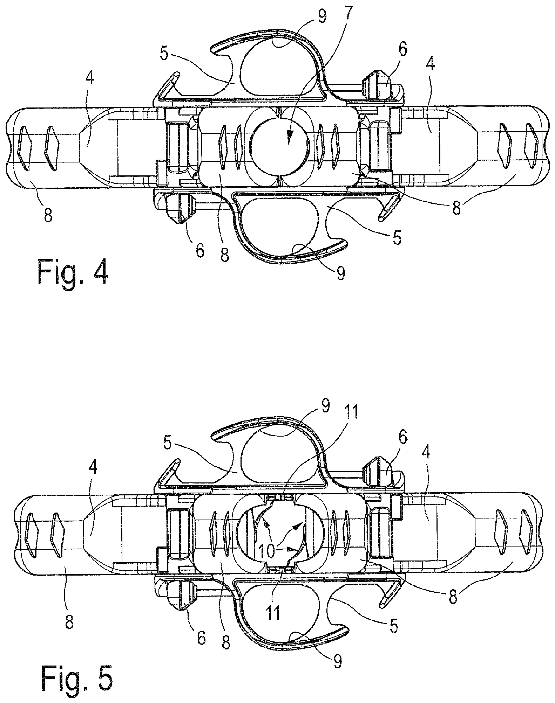

Bei der praktischen Ausführungsform gemäß den Fig. 4 bis 15 hingegen ist die Synchronisiereinrichtung gezeigt. Diese wird gebildet durch entsprechende Verzahnungssegmente 11, die an dem jeweiligen Klemmglied 4 angeformt und koaxial zu der jeweiligen Drehachse 6 des Klemmgliedes 4 ausgerichtet sind. Die Verzahnungssegmente 11 erstecken sich kreisbogenförmig über einen Winkel von etwa 90° relativ zur Drehachse 6. Die Verzahnungssegmente 11 der benachbarten Klemmglieder 4 kämmen miteinander.

Anhand der Fig. 4 bis 15 ist erkennbar, dass jedes Klemmglied 4 als einstückiges Kunststoffbauteil gestaltet ist, das um die jeweilige Drehachse 6 drehbar gelagert ist. Das Funktionsprinzip der Durchflussregeleinrichtung gemäß den Fig. 4 bis 15 entspricht dem zuvor beschriebenen Funktionsprinzip gemäß den Fig. 1 bis 3. Die Drehachsen 6 entsprechen demzufolge funktional den Drehachsen D, die Klemmglieder 4 entsprechen den Klemmgliedern 1 gemäß den Fig. 1 bis 3 und entsprechende exzentrische Klemmabschnitte 10 entsprechen den exzentrischen Klemmabschnitten 2, 3, wie sie anhand der Fig. 1 bis 3 bereits erläutert wurden. Nachfolgend wird lediglich noch auf die Ausführungsform gemäß den Fig. 4 bis 15 eingegangen. Jedes Klemmglied 4 bildet einen Klemmkörper, der auf einer zum gegenüberliegenden Klemmglied 4 gewandten Innenseite mit dem exzentrischen Klemmabschnitt 10 mit entsprechenden Anlagekonturen für den zu quetschenden Schlauchabschnitt versehen ist. Die Anlagekonturen der exzentrischen Klemmabschnitte 10 sind anhand der Fig. 5 bis 7 erkennbar. Die exzentrischen Klemmabschnitte 10 erstrecken sich - wie auch die Verzahnungssegmente 11 - über einen Umfangswinkel von etwa 90° relativ zur jeweiligen Drehachse 6. Die exzentrischen Klemmabschnitte 10 sind zwischen den randseitigen Verzahnungssegmenten 11 vorgesehen. Anhand der Fig. 5 bis 7 ist erkennbar, dass jedes Klemmglied 4 als einteiliges Schalenbauteil gestaltet ist, das im Bereich seiner gegenüberliegenden Seitenwände randseitig mit jeweils einem Verzahnungssegment 11 versehen ist, so dass die beiden Klemmglieder 4 über insgesamt vier Verzahnungssegmente 11 miteinander kämmen. Zwischen den seitlichen Verzahnungssegmenten 11 ist jeweils der exzentrische Klemmabschnitt 10 des jeweiligen Klemmgliedes 4 vorgesehen. Die beiden Klemmglieder 4 sind identisch zueinander gestaltet, jedoch um 180° verdreht zueinander positioniert. Die beiden Klemmglieder 4 bilden demzufolge Gleichteile. Die beiden Klemmglieder 4 sind mit ihren Drehachsen 6 in einem Trägergehäuse drehbar gelagert, das eine Bodenschale und eine Deckschale 5 bildet, die ebenfalls als Gleichteile ausgeführt sind. Die Bodenschale und die Deckschale 5 sind mit einer einstückig angeformten Greifhandhabe 9 versehen. Die beiden Gehäuseschalen 5 des Trägergehäuses sind über entsprechende Steckelemente im Bereich der beiden Drehachsen 6 miteinander verbunden. Die Steckelemente sind zum einen als Rastelemente zum Sichern der beiden Gehäuseschalen 5 gegeneinander und zum anderen als Drehachsen für die Klemmglieder 4 ausgeführt, die entsprechend komplementäre Lageraufnahmen bilden. Wenigstens ein Steckelement jeder Drehachse 6 ist als Steckstift ausgeführt, um eine Montage und gegebenenfalls eine Demontage der Gehäuseschalen 5 und der Klemmglieder 4 relativ zueinander zu ermöglichen. Der Steckstift kann einstückig an der Deckschale bzw. der Bodenschale angeformt sein.

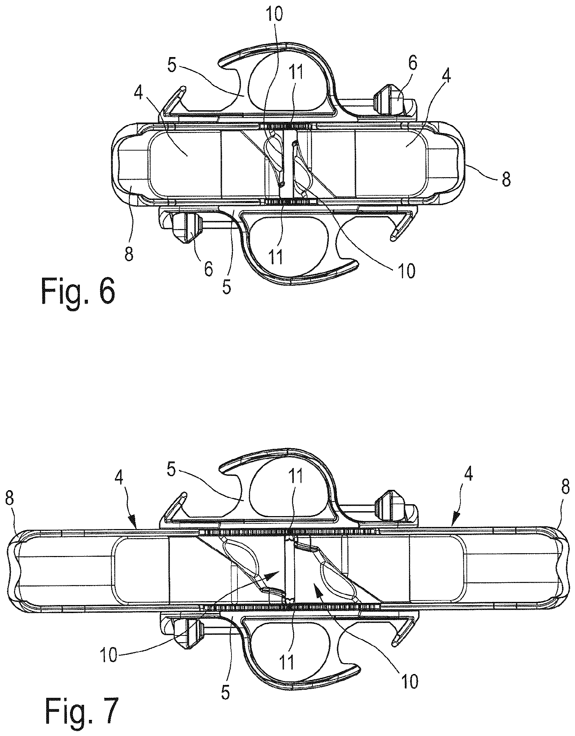

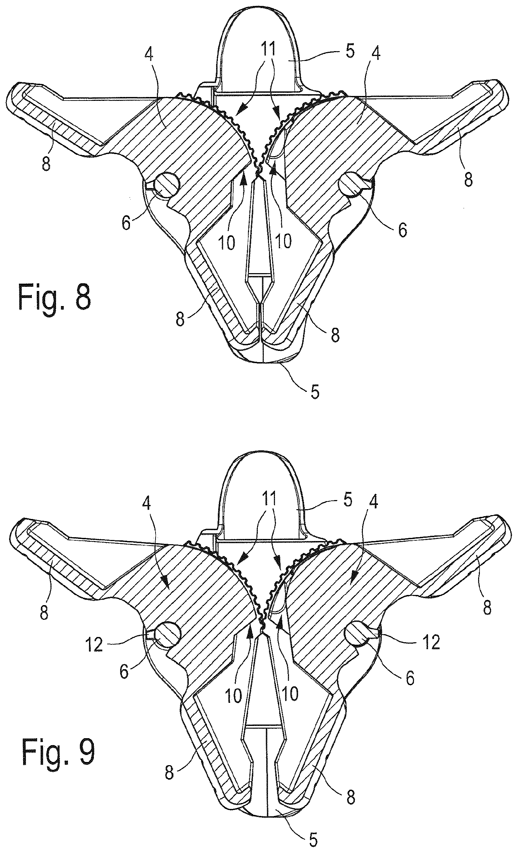

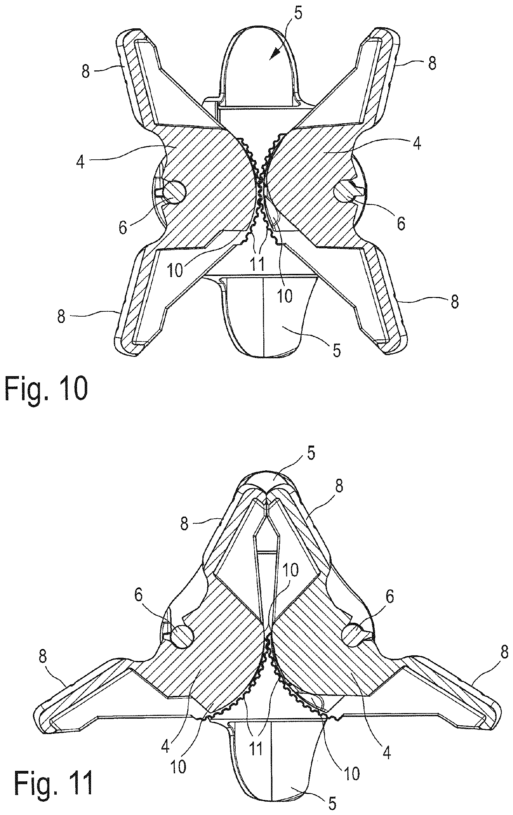

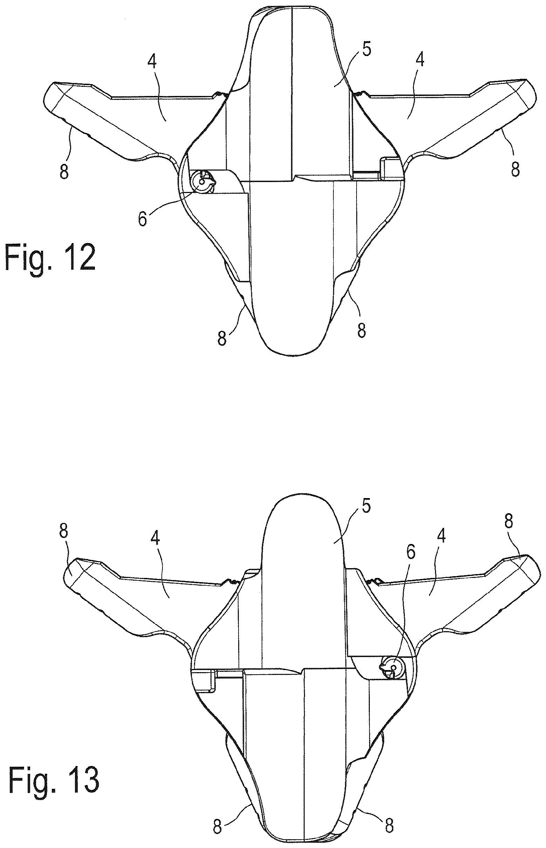

Anhand der Fig. 8 bis 11 ist erkennbar, dass die Drehachsen 6 mit Anschlägen 12 versehen sind, die eine Drehbeweglichkeit des jeweiligen Klemmgliedes 4 auf einen Winkel von etwa 90° begrenzen. Die Lageraufnahmen der Klemmglieder 4 weisen entsprechende komplementäre Anschläge auf, um die begrenzte Drehbeweglichkeit sicherzustellen. Jedes Klemmglied 4 weist zwei flügelförmige Bedienhebel 8 auf, die einstückig an dem Klemmkörper angeformt sind und relativ zur Drehachse 6 zu gegenüberliegenden Seiten hin abragen. Auch die flügelförmigen Bedienhebel 8 sind in der Radialebene angeordnet, in der sich das jeweilige Klemmglied 4 erstreckt. Die flügelförmigen Bedienhebel 8 ragen relativ zum jeweiligen exzentrischen Klemmabschnitt 10 von der Drehachse 6 zu gegenüberliegenden Seiten hin ab, um bei einer manuellen Betätigung entsprechende Hebelarme für eine Verdrehung des jeweiligen Klemmgliedes 4 zu bilden. Anhand der Fig. 8 bis 11 ist der maximale Stellweg der beiden Klemmglieder 4 erkennbar. In Fig. 8 ist eine Montagestellung zur Montage der Durchflussregeleinrichtung auf einer entsprechenden Schlauchleitung dargestellt. In Fig. 9 ist eine weitgehend offene Stellung der Durchflussregeleinrichtung gemäß Fig. 5, in Fig. 10 eine weitgehend geschlossene Stellung entsprechend Fig. 6 und in Fig. 11 eine vollkommen geschlossene Stellung gemäß Fig. 7 gezeigt. Die Fig. 12 bis 15 zeigen die Durchflussregeleinrichtung gemäß den Fig. 8 bis 11 in einer Draufsicht in jeweils analogen Funktionsstellungen, wobei Fig. 12 eine in der Zeichnungsebene um 180° gegenüber der Darstellung nach Fig. 13 verdrehte Ausrichtung zeigt.

Anhand der Fig. 8 bis 15 ist erkennbar, dass die beiden Klemmglieder 4 spiegelsymmetrisch zu einer Längserstreckung eines lediglich anhand der Fig. 1 bis 3 erkennbaren Schlauchabschnittes S synchron und gegenläufig zueinander verstellbar sind. Anhand der Fig. 8 bis 15 ist auch erkennbar, dass immer wenigstens zwei flügelförmige Bedienhebel 8 der Klemmglieder 4 seitlich über außenseitige Randkonturen der Gehäuseschalen 5 hinausragen, so dass wenigstens ein Bedienhebel 8 in einfacher Weise ergriffen und bedient werden kann. Vorzugsweise greift eine Bedienperson mit dem Finger einer Hand in die Greifhandhabe 9 einer Gehäuseschale 5 ein und drückt mit einem anderen Finger, vorzugsweise mit dem Daumen, einen der flügelförmigen Bedienhebel 8 in eine entsprechend gewünschte Drehrichtung. Hierdurch wird zwangsläufig eine entsprechende Durchflussrate des Schlauchabschnittes S verändert. Da der gesamte Verstellwinkel der beiden Klemmglieder 4 insgesamt nur etwa 90° beträgt, muss die Bedienperson nicht umgreifen, um die Schlauchleitung aus einer vollkommen offenen Stellung vollständig abzusperren. Zudem ist die Bedienung gleichermaßen für Rechtsund für Linkshänder geeignet.

Die Steckelemente im Bereich der Drehachsen 6 wie auch die Klemmglieder 4 und die Gehäuseschalen 5 sind derart gestaltet, dass die einzelnen Bauteile werkzeuglos zu der betriebsfertigen Baueinheit zusammengefügt werden können, die die Durchflussregeleinrichtung gemäß den Fig. 4 bis 15 darstellt. Vorzugsweise wird die jeweilige Drehachse 6 durch jeweils einen Steckstift gebildet, der innenseitig an jeweils einer Gehäuseschale 5 einstückig angeformt ist. In Verbindung mit den Lageraufnahmen im Bereich der Klemmglieder 4 und entsprechenden, nicht näher bezeichneten Durchtritten im Bereich der jeweiligen gegenüberliegenden Gehäuseschale 5 wird die Durchflussregeleinrichtung gemäß den Fig. 4 bis 15 demzufolge durch lediglich vier Bauteile gebildet, nämlich die beiden Gehäuseschalen 5 und die beiden Klemmglieder 4, an denen die Verzahnungssegmente 11 und die flügelförmigen Bedienhebel 8 jeweils angeformt sind.

## Patentansprüche

1. Durchflussregeleinrichtung für eine Schlauchleitung eines medizinischen Infusionssystems, mit wenigstens einem drehbeweglichen Klemmglied (4), das in montiertem Zustand mit der Schlauchleitung in Wirkverbindung ist, und das manuell relativ zu einem Schlauchabschnitt (S) der Schlauchleitung verstellbar ist, um eine Fluiddurchflussrate des Schlauchabschnittes (S) zu verändern, wobei zwei drehbeweglich gelagerte Klemmglieder (1, 4) vorgesehen sind, zwischen denen der Schlauchabschnitt (S) hindurchgeführt ist und die einen Mantel des Schlauchabschnittes (S) mit ihren Umfangskonturen auf gegenüberliegenden Seiten flankieren, und wobei die Umfangskontur wenigstens eines Klemmgliedes (1, 4) zumindest abschnittsweise exzentrisch zu einer Drehachse (D, 6) des Klemmgliedes (1, 4) gestaltet ist, wobei die beiden Klemmglieder (4) mittels einer Synchronisiereinrichtung synchron gegenläufig zueinander drehbeweglich gelagert sind und wobei die Synchronisiereinrichtung zwei miteinander kämmende, kreisbogenförmige Verzahnungsabschnitte (Verzahnungssegmente 11) aufweist, die jeweils einem Klemmglied (4) zugeordnet sind,
**dadurch gekennzeichnet, dass** ein exzentrischer Klemmabschnitt (10) der Umfangskontur gekrümmt ausgeführt ist mit einem radialen sowie einem axialen Stützwandungsbereich (2, 3), die den Schlauchabschnitt (S) relativ zu einer Drehachse (D, 6) des Klemmgliedes (1, 4) mantelseitig beaufschlagen abhängig von einer Drehung des Klemmgliedes (1, 4).

2. Durchflussregeleinrichtung nach Anspruch 1, **dadurch gekennzeichnet, dass** die Verzahnungsabschnitte (11) koaxial zu der jeweiligen Drehachse (6) ausgerichtet sind.

3. Durchflussregeleinrichtung nach Anspruch 1 oder 2, **dadurch gekennzeichnet, dass** die Verzahnungsabschnitte (11) einstückig an den Klemmgliedern (4) vorgesehen sind.

4. Durchflussregeleinrichtung nach einem der vorhergehenden Ansprüche, **dadurch gekennzeichnet, dass** ein Verstellwinkel jedes Klemmgliedes (4) zwischen einer den Schlauchabschnitt (S) für einen vollen Fluiddurchfluss freigebenden Öffnungsstellung und einer den Schlauchabschnitt (S) für einen Fluiddurchfluss verschließenden Sperrstellung kleiner oder gleich 90° ist.

5. Durchflussregeleinrichtung nach Anspruch 4, **dadurch gekennzeichnet, dass** ein durch den jeweiligen kreisbogenförmigen Verzahnungsabschnitt (11) überstrichener Winkel maximal 90° beträgt.

6. Durchflussregeleinrichtung nach einem der vorhergehenden Ansprüche, **dadurch gekennzeichnet, dass** die beiden Klemmglieder (4) relativ zueinander gelagert und gehalten sind, und/oder dass insbesondere die beiden Klemmglieder (4) als Gleichteile gestaltet sind, und/oder dass insbesondere jedes Klemmglied (4) wenigstens einen Bedienhebel (8) aufweist, der gegenüberliegend zu dem exzentrischen Klemmabschnitt (10) relativ zu der Drehachse (6) nach außen abragt, und/oder dass insbesondere jedes Klemmglied (4) zwei Bedienhebel (8) aufweist, die wippenartig in unterschiedlichen Richtungen zu der Drehachse (6) abragen, und/oder dass insbesondere der wenigstens eine Bedienhebel (8) zumindest abschnittsweise seitlich nach außen über das Trägergehäuse hinausragt.

7. Durchflussregeleinrichtung nach Anspruch 6, **dadurch gekennzeichnet, dass** die beiden Klemmglieder (4) in einem Trägergehäuse (5) drehbar gelagert sind.

8. Durchflussregeleinrichtung nach Anspruch 7, **dadurch gekennzeichnet, dass** das Trägergehäuse aus einer Bodenschale (5) und einer Deckschale (5) aufgebaut ist, zwischen denen die Klemmglieder (4) angeordnet sind, wobei die Bodenschale und die Deckschale im Bereich der Drehachsen (6) miteinander verbunden sind.

## Claims

1. Through-flow regulating device for a hose line of a medical infusion system, having at least one rotationally movable clamping member (4) which, in the mounted state, is operatively connected to the hose line and which can be manually adjusted relative to a hose section (S) of the hose line in order to change a fluid through-flow rate of the hose section (S), wherein two clamping members (1, 4) mounted in a rotationally movable manner are provided, between which the hose section (S) is passed through and which flank a jacket of the hose section (S) on opposite sides with their circumferential contours, and wherein the circumferential contour of at least one clamping member (1, 4) is formed, at least in sections, in an eccentric manner relative to an axis of rotation (D, 6) of the clamping member (1, 4), wherein the two clamping members (4) are mounted in a rotationally movable manner, such that they move synchronously in opposite directions to one another, by means of a synchronizing device, and wherein the synchronizing device has two circular-arc-shaped toothing sections (toothing segments 11) which mesh with one another and are in each case assigned to a clamping member (4),
**characterized in that**
an eccentric clamping section (10) of the circumferential contour is of curved design with a radial and an axial supporting wall region (2, 3), which regions act upon the hose section (S) on the jacket side relative to an axis of rotation (D, 6) of the clamping member (1, 4) in dependence on a rotation of the clamping member (1, 4).

2. Through-flow regulating device according to claim 1, **characterized in that** the toothing sections (11) are oriented co-axially relative to the respective axis of rotation (6).

3. Through-flow regulating device according to claim 1 or 2, **characterized in that** the toothing sections (11) are provided in one piece on the clamping members (4).

4. Through-flow regulating device according to any of the preceding claims, **characterized in that** an adjustment angle of each clamping member (4) between an open position, in which it opens up the hose section (S) for a full fluid through-flow, and a blocked position, in which it closes the hose section (S) for a fluid through-flow, is less than or equal to 90°.

5. Through-flow regulating device according to claim 4, **characterized in that** an angle covered by the respective circular-arc-shaped toothing section (11) is at most 90°.

6. Through-flow regulating device according to any of the preceding claims, **characterized in that** the two clamping members (4) are mounted and held relative to one another, and/or **in that** in particular the two clamping members (4) are formed as identical parts, and/or **in that** in particular each clamping member (4) has at least one operating lever (8) which projects outwardly relative to the axis of rotation (6), opposite to the eccentric clamping section (10), and/or **in that** in particular each clamping member (4) has two operating levers (8) which project in the manner of a rocker in different directions relative to the axis of rotation (6), and/or **in that** in particular the at least one operating lever (8) projects laterally outwards beyond the carrier housing at least in sections.

7. Through-flow regulating device according to claim 6, **characterized in that** the two clamping members (4) are rotatably mounted in a carrier housing (5).

8. Through-flow regulating device according to claim 7, **characterized in that** the carrier housing is constructed from a base shell (5) and a cover shell (5), between which the clamping members (4) are arranged, wherein the base shell and the cover shell are connected to one another in the region of the axes of rotation (6).

## Revendications

1. Dispositif de régulation de débit destiné à une conduite en tuyaux souples d'un système de perfusion médical, ledit dispositif comprenant au moins un élément de serrage rotatif (4) qui est relié fonctionnellement à la conduite en tuyaux souples à l'état monté et qui est réglable manuellement par rapport à une portion de tuyau souple (S) de la conduite en tuyaux souples pour faire varier le débit de fluide de la portion de tuyau souple (S), deux éléments de serrage (1, 4) montés à rotation étant prévus entre lesquels la portion de tuyau souple (S) est guidée et qui flanquent une enveloppe de la portion de tuyau souple (S) de leurs contours périphériques sur des côtés opposés, et le contour périphérique d'au moins un élément de serrage (1, 4) étant au moins partiellement excentré par rapport à un axe de rotation (D, 6) de l'élément de serrage (1, 4), les deux éléments de serrage (4) étant montés à rotation de manière synchrone dans des sens opposés l'un à l'autre au moyen d'un dispositif de synchronisation et le dispositif de synchronisation comportant deux portions de denture en forme d'arc de cercle (segments de denture 11) qui s'engrènent l'une avec l'autre et qui sont chacune associées à un élément de serrage (4), **caractérisé en ce qu'**une portion de serrage excentrique (10) du contour périphérique est incurvée et comporte des régions de paroi de support radiale et axiale (2, 3) qui sollicitent la portion de tuyau souple (S) par rapport à un axe de rotation (D, 6) de l'élément de serrage (1, 4) sur le côté enveloppe en fonction de la rotation de l'élément de serrage (1, 4).

2. Dispositif de régulation de débit selon la revendication 1, **caractérisé en ce que** les portions de denture (11) sont orientées coaxialement à l'axe de rotation respectif (6).

3. Dispositif de régulation de débit selon la revendication 1 ou 2, **caractérisé en ce que** les portions de denture (11) sont prévues d'une seule pièce sur les éléments de serrage (4).

4. Dispositif de régulation de débit selon l'une des revendications précédentes, **caractérisé en ce qu'**un angle de réglage de chaque élément de serrage (4), formé entre une position ouverte libérant la portion de tuyau souple (S) destinée à un écoulement de fluide complet et une position de blocage fermant la portion de tuyau souple (S) destinée à un écoulement de fluide, est inférieur ou égal à 90°.

5. Dispositif de régulation de débit selon la revendication 4, **caractérisé en ce qu'**un angle, balayé par la portion de denture respective (11) en forme d'arc de cercle est de 90° maximum.

6. Dispositif de régulation de débit selon l'une des revendications précédentes, **caractérisé en ce que** les deux éléments de serrage (4) sont montés et maintenus l'un par rapport à l'autre et/ou **en ce que** notamment les deux éléments de serrage (4) sont identiques et/ou **en ce que**, en particulier, chaque élément de serrage (4) comporte au moins un levier de commande (8) qui fait saillie vers l'extérieur à l'opposé de la portion de serrage excentrique (10) par rapport à l'axe de rotation (6) et/ou **en ce que**, en particulier, chaque élément de serrage (4) comporte deux leviers de commande (8) qui font saillie à la manière d'une bascule dans des directions différentes par rapport à l'axe de rotation (6) et/ou **en ce que**, en particulier, l'au moins un levier de commande (8) fait saillie au moins par endroits latéralement vers l'extérieur sur le boîtier porteur.

7. Dispositif de régulation de débit selon la revendication 6, **caractérisé en ce que** les deux éléments de serrage (4) sont montés à rotation dans un boîtier porteur (5).

8. Dispositif de régulation de débit selon la revendication 7, **caractérisé en ce que** le boîtier porteur est réalisé à partir d'une coque de fond (5) et d'une coque de couvercle (5) entre lesquelles sont disposés les éléments de serrage (4), la coque de fond et la coque de couvercle étant reliées l'une à l'autre au niveau des axes de rotation (6).
